**Europäisches Patentamt**

**European Patent Office**

**Office européen des brevets**

(19)

(11) Veröffentlichungsnummer : **0 379 753 B1**

(12)

# EUROPÄISCHE PATENTSCHRIFT

(45) Veröffentlichungstag der Patentschrift :
**11.11.92 Patentblatt 92/46**

(51) Int. Cl.⁵ : **A61K 31/70, A61K 9/20**

(21) Anmeldenummer : **89250020.8**

(22) Anmeldetag : **28.08.89**

(54) **N-Acetylglucosamin-Zubereitungen zur buccalen Anwendung.**

Die Akte enthält technische Angaben, die nach dem Eingang der Anmeldung eingereicht wurden und die nicht in dieser Patentschrift enthalten sind.

(30) Priorität : **26.01.89 US 302403**

(43) Veröffentlichungstag der Anmeldung :
**01.08.90 Patentblatt 90/31**

(45) Bekanntmachung des Hinweises auf die
Patenterteilung :
**11.11.92 Patentblatt 92/46**

(84) Benannte Vertragsstaaten :
**AT BE CH DE ES FR GB GR IT LI LU NL SE**

(56) Entgegenhaltungen :
**EP-A- 0 178 602**
**DE-A- 3 602 670**
**FR-A- 2 016 182**
**Remington's Pharmac.Sciences, 15th**
**ed.,1975, p. 683-685**

(73) Patentinhaber : **STEIGERWALD**
**ARZNEIMITTELWERK GMBH**
**Havel Strasse 5**
**W-6100 Darmstadt (DE)**

(72) Erfinder : **Speck, Ulrich, Dr.**
**Benediktiner Strasse 50**
**W-1000 Berlin 28 (DE)**

(74) Vertreter : **Wablat, Wolfgang, Dr.Dr.**
**Potsdamer Chaussee 48**
**W-1000 Berlin 38 (DE)**

## Beschreibung

Die Gelenke des Menschen und der Wirbeltiere sind außerordentlich starken, wechselhaften und z. T. zivilisationsbedingt einseitigen Belastungen ausgesetzt. Sehr glatte Oberflächen der Knochen an den Gelenkflächen, die hervorragende Schmierfähigkeit der Synovialflüssigkeit, sowie elastische, jedoch mechanisch stark belastbare Knorpel und Bänder gewährleisten vor allem in der Jugend eine einwandfreie Funktion der Gelenke. Bereits im mittleren Lebensalter sind degenerative Prozesse an den am meisten belasteten Gelenken, den Knien und Hüften und der Wirbelsäule zu beobachten, die in vielen Fällen auch zu klinisch relevanten Beschwerden führen. Solche Veränderungen betreffen zunächst vor allem die Qualität der Synovialflüssigkeit und der Knorpel, in späteren Stadien sind Aufrauhungen und Erosionen an den Knochen selbst zu beobachten. Schmerzen und Einschränkungen der Bewegungsfähigkeit bis hin zur vollständigen Versteifung der Gelenke können die Folge sein.

Der Prozeß der Gelenkschädigung kann durch viele äußere Einflüsse verstärkt werden,: Tragen schwerer Lasten, ungünstige Körperhaltung, völliger Mangel an Bewegung, exzessiver Sport etc. Weiterhin können falsche Ernährung, Stoffwechselkrankheiten, Infektionen, rheumatische Erkrankungen etc. zu einem raschen Fortschreiten Regenerativer Gelenkerkrankungen beitragen oder diese in Gang bringen.
In höherem Lebensalter bleibt kaum jemand von entsprechenden Beschwerden verschont.

Die Probleme bei der Behandlung degenerativer Gelenkveränderungen sind vielfältig: Der Beginn der Krankheit wird nicht erkannt. Beim Auftreten erster Beschwerden liegen oft schon kaum noch reversible Veränderungen vor. Die Ursachen variieren, der Mechanismus der Knorpeldegeneration und anderer pathologischer Prozesse ist meist nicht bekannt. Eine kausale Therapie ist selten möglich oder setzt zu spät ein.

Die Therapie der schmerzhaften, teils auch endzündlichen Zustände erfolgt häufig nur symptomatisch mit Hilfe von nicht-steroidalen Entzündungshemmern, wie beispielsweise Indomethacin oder sogar durch Einsatz von Kortikoiden. Beide Gruppen von Therapeutika verursachen gravierende Nebemwirkungen und sollten daher so wenig wie möglich eingesetzt werden. Darüber hinaus besteht bei der Anwendung der nichtsteroidalen Entzündungshemmer und bei den Kortikoiden die Gefahr einer weiteren Verschiebung des Stoffwechsels der Glycosaminoglycane (GAGs) in Richtung auf einen beschleunigten Abbau. Dem Vorteil der momentanen Linderung der Symptome der Erkrankung, wie Schmerz und Unbeweglichkeit der Gelenke, steht daher neben anderen Risiken die Gefahr einer Beschleunigung der degenerativen Prozesse, welche die Krankheit verursachen, gegenüber.

Sei langem ist bekannt, daß demgegenüber Glucosaminoglycane oder auch die Vorstufe eines Bausteins der GAGs, das Glucosamin, eine ursächlich therapeutische Wirkung ausüben. Die Wirkung beruht einerseits auf einem Einbau der betreffenden Bausteine in die GAGs, andererseits in einer Stimulierung der Neusynthese von GAGs durch eine Erhöhung der Konzentration von Vorstufen ihrer Synthese. Damit besteht die Möglichkeit, die für die Erkrankung ursächlichen Stoffwechselprozesse günstig zu beeinflussen und damit zu einer Heilung oder zumindenstens Verlangsamung der degenerativen Vorgänge, welche der Erkrankung zugrunde liegen, beizutragen.

Nun sind allerdings die für die zuletzt erwähnte ursächliche Therapie zur Verfügung stehenden Arzneistoffe ebenfalls noch nicht ideal.

Aus biologischem Material isolierte GAGs weisen den Nachteil komplexer Naturprodukte auf: Sie sind nur schwer oder kaum eindeutig zu definieren; ihre parenterale Anwendung ist notwendig, um eine ausreichende Bioverfügbarkeit zu gewährleisten, andererseits aber bei der Langzeittherapie doch unerwünscht. Daneben besteht immer die Gefahr anaphylaktischer Reaktionen. Die begrenzte Löslichkeit und die hohe Viskosität konzentrierter Lösungen erschweren die Verabreichung in der wünschenswert hohen Dosierung.

Anstelle der natürlichen GAGs wurde mit gutem therapeutischen Erfolg auch Glucosaminsulfat oral, intramuskulär und intraartikulär verabreicht. Glucosaminsulfat hat den großen Vorteil, eine im Hinblick auf Identität, Reinheit und Stabilität eindeutig definierbare Verbindung zu sein. Glucosaminsulfat verursacht als niedermolekulare, natürliche Substanz keine Allergien und läßt in der notwendigen Dosierung kaum toxische Wirkungen erwarten. Andererseits weist auch Glucosaminsulfat wesentliche Nachteile auf, wie sie sich beispielsweise aus der Basisinformation Dona®200-S der Firma Opfermann-Arzneimittel, 5060 Bergisch Gladbach 2, ablesen lassen:

Die orale Applikationsform ist offenbar sehr viel weniger wirksam als die intravenöse oder intramuskuläre Injektion. Es wird eine orale Wochendosis von 5250 mg empfohlen, wogegen parenteral nur 1200 mg notwendig sind. Das wirksamere Injektionspräparat ist in Lösung bei physiologischem pH-Wert nicht ausreichend stabil; es wird daher bei saurem pH-Wert zubereitet, gelagert und geliefert, und muß vor Gebrauch vom Arzt neutralisiert werden. Zu diesem Zweck wird der Glucosaminsulfatlösung eine Pufferlösung zugesetzt. Glucosaminsulfatlösung und Puffer haben insgesamt bei der notwendigen hohen Dosierung und Konzentration einen gegenüber dem Blut so hohen osmotischen Druck, daß zusätzlich Lidocain als Lokalanästhetikum zugesetzt werden muß. Erst durch diesen Zusatz wird die Injektion in die Gelenke ausreichend schmerzarm.

Dem Nachteil der zu geringen Wirksamkeit oral verabreichten Glucosamins und der geringen chemischen Stabilität wurde versucht, durch die Verwendung spezifischer Salze und Salzgemische entgegenzuwirken. So konnte die Wirksamkeit von Glucosamin durch den Einsatz von Gemischen des Sulfates und Hydrojodids etwas verbessert werden (Rovati, 1968, US Patent 36 83 076). Senin et al., 1981 erzeugten spezielle Mischkristalle aus NaCl und Glucosaminsulfat, die besonders wenig hygroskopisch und ausreichend stabil sein sollen (DOS 32 15 844 A 1). Der Geschmack wird allerdings als sehr bitter angegeben.

Zur Überwindung der Stabilitätsprobleme mit den Glucosaminsalzen schlugen Rovati et al. 1968 vor, beispielsweise N-Acetylglucosamin zu verwenden (US Patent 36 97 652, DE 17 92 346 C 3). Um die Wirksamkeit zu steigern, wurden

a) wäßrige Lösungen zur Injektion bevorzugt und/oder

b) dem N-Acetylglucosamin die Salze $Na_2O_4$ und NaJ zugesetzt.

Meisner, 1985, empfahl sogar den Zusatz von 3 unterschiedlichen Stoffklassen, um eine ausreichende Wirkung von oral verabreichten Aminozuckern einschließlich N-Acetylglucosamin zur Vorbeugung oder Behandlung degenerativ-entzündlicher Erkrankungen zu erzielen (US Patent 46 47 453).

Aus der DE-A-3 602 670 ist die Verwendung von N-Acetylglucosamin zur Herstellung von Arzneimitteln zur Behandlung degenerativer Erkrankungen der Gelenke und des Binde- und Stützgewebes sowie verwandter Erkrankungen bekannt. Diese Arzneimittel können als wäßrige Lösung zu Injektionen und Infusionen oder in fester Form als Pulver, Granulate, Tabletten oder Dragees zur oralen Einnahme vorliegen. Durch Verwendung von N-Acetylglucosamin anstelle von Glucosaminsulfat kommt es bei oraler wie parenteraler Gabe zu einem besseren Einbau. Es wird aber nur die konventionelle orale Applikationsform beschrieben. Pulver, Granulate, Kapseln, Tabletten und Dragees werden als gleichwertig nebeneinandergestellt, wodurch klar ist, daß die übliche Einnahme oraler Dosisformen durch Verschlucken und Nachspülen mit Flüssigkeit angesprochen ist.

Es ist deutlich erkenntlich, daß trotz sehr hohen Bedarfes bisher keine optimalen Arzneimittel zur Behandlung degenerativer und/oder entzündlicher Gelenkerkrankungen bzw. der Erkrankungen anderer Binde- und Stützgewebe verfügbar sind.

Die Verwendung von Aminozuckern zur Behandlung derartiger Erkrankungen bietet große Vorteile, weil sie im Gegensatz zu den Korticoiden und gebräuchlichen nichtsteroidalen Entzündungshemmern ausschließlich aufbauende, die Synthese der Glycosaminoglycane fördernde Wirkungen entfalten.

Es handelt sich um eindeutig definierte, nicht antigen wirksame, ausgezeichnet verträgliche Substanzen. Bei Einsatz des N-Acetylglucosamins anstelle des Glucosamins gibt es kaum Stabilitätsprobleme bei der Langzeitlagerung.

Bisher liegt aber keiner der Aminozucker in einer ausreichend wirksamen Form vor, um als alleiniger Wirkstoff eines vom Patienten leicht selbst anzuwendenden Präparates eingesetzt zu werden.

Injektionsformen sind für die Dauertherapie unerwünscht, weil sie häufige und damit sehr teure Besuche beim Arzt notwendig machen. Darüber hinaus sind Injektionspräparate per se teuer und für den Patienten unangenehm.

Kombination mehrerer Wirkstoffe miteinander sind ebenfalls unerwünscht:

a) Die Stabilität jeder Einzelkomponente unter den Bedingungen der Langzeitlagerung ist schwierig zu gewährleisten.

b) Aus gutem Grund verhalten sich die Gesundheitsbehörden vieler Länder bei der Zulassung von Kombinationspräparaten sehr restriktiv. So wird der Nachweis verlangt, daß jeder einzelne Inhaltsstoff des Kombinationspräparates wirksam ist und häufig zusätzlich, daß die feste Kombination eines bestimmten Mengenverhältnisses der Einzelkomponenten spezifische Vorteile hat. Diese Belege sind für Kombinationen aus mehr als 2 Wirkstoffen kaum zu erbringen.

Von den Aminozuckern hat N-Acetylgrlucosamin den Vorteil, stabil und zumidest nach parenteraler Gabe zweifelsfrei gegen degenerative Gelenkerkrankungen wirksam zu sein.

Gegenstand der Erfindung ist nun die Verwendung von N-Acetylglucosamin zur Herstellung eines buccal anzuwendenden Arzneimittels für die Therapie degenerativer Erkrankungen der Gelenke und des Binde- und Stützgewebes sowie verwandter Erkrankungen. Besonders bevorzugte Ausführungsformen der erfindungsgemäßen Verwendung von N-Acetylglucosamin zur Herstellung eines buccal anzuwendenden Arzneimittels zeichnen sich dadurch aus, daß

- N-Acetylglucosamin in fester, halbfester oder flüssiger Form zusammen mit pharmazeutisch unbedenklichen Lösungs- , Träger- und/oder Hilfsstoffen eingesetzt wird,

- N-Acetylglucosamin in fester Form als Pulver, Granulat, Tabletten, elastische oder plastische Kaumaterialien eingesetzt wird,

- N-Acetylglucosamin unter Zusatz unbedenklicher und üblicher Geschmacksstoffe, Aromastoffe, pharmazeutischer Träger- und Hilfsstoffe zur Stabilisierung, Formgebung und Steuerung der Freigabe eingesetzt wird.

Die Wirksamkeit von N-Acetylglucosamin läßt sich auch nach oraler Gabe durch einen spezifischen Einnahmemodus steigern. Im Gegensatz zu Glucosamin hat N-Acetylglucosamin einen überraschend guten, nicht zu starken rein süßen Geschmack. Trotz des relativ hohen Molekulargewichtes und des sehr hydrophilen Charakters des Moleküls kommt es bereits in der Mundhöhle zur Resorption eines Teils des Wirksstoffes. Dadurch wird der Abbau in der Darmucosa und der Leber vor Erreichen des allgemeinen Kreislaufes vermieden. Im Ergebnis ist N-Acetylglucosamin bei oraler Einnahme besser bioverfügbar und wirksam, wenn es möglichst lange Zeit im Mund behalten wird und mit den Schleimhäuten im Kontakt bleibt. Dies kann auf sehr einfache Weise erreicht werden: Bereits bei Einnahme des N-Acetylglucosamins in Form von Pulver oder Granulat kann ein Teil in der Mundhöhle resorbiert werden, wenn nicht sofort mit Flüssigkeit nachgespült wird.

Sehr bequem ist die Einnahme als Lutschtablette mit fester Dosierung oder als Zusatz zu Materialien zum Kauen. Gute Wirksamkeit, angenehmer Geschmack, die Vermeidung aufwendiger und invasiver Injektionsformen und selbst des Schluckens großer Tabletten machen die neue Applikationsform zum idealen Mittel für die notwendige Langzeittherapie degenerativer Gelenkerkrankungen. Weitere Vorteile sind darin zu sehen, daß N-Acetylglucosamin sehr gut verträglich ist und der Abbau dieses Naturstoffes im Stoffwechsel auch unter ungünstigen Voraussetzungen bei alten Patienten keine Probleme bereitet.

N-Acetylglucosamin zur buccalen Verabreichung kann in einer Tagesdosis von 50 mg bis 1000 mg eingesetzt werden. Höhere Dosierungen sind wegen der sehr guten Verträglichkeit möglich, bevorzugt sind 200 mg bis 600 mg pro Tag.

Die Einzeldosis kann bei mehrfacher Einnahme pro Tag 10 mg bis 500 mg N-Acetylglucosamin enthalten, bevorzugt sind 50 mg bis 250 mg.

Geeignet sind alle Zubereitungsformen, die den Wirkstoff bereits im Mund freisetzen, den angenehmen Geschmack des N-Acetylglusosamins nicht verschlechtern, und keine Zusatzstoffe enthalten, die entweder physiologisch unverträglich oder in der Konsistenz unangenehm sind. Bevorzgut sind Zubereitungen, die den Wirkstoff beim Lutschen oder Kauen langsam freisetzen und sich selbt gleichzeitig auflösen.

Diese Ziel kan entweder durch geeignete Zubereitungen des N-Acetylglucosamin selbst oder durch Zusatz üblicher pharmazeutischer Hilfsstoffe erreicht werden. Geeignete Hilfsstoffe sind z. B. Zucker und verwandte Stoffe (Saccharose, Lactose, Glucose, Mannit, Sorbit, Fructose etc.), Quellstoffe (Agar, Gummiarabicum, Guar, Gelatine etc.), Stärke, Dextrane, Dextrine, Geschmacks- und Aromastoffe (Zitronensäure, Weinsäure, Ascorbinsäure, Pfefferminzöl etc.), Puffer-Salze, Lösungsvermittler.

Als Formen eignen sich Pulver, Granulate und bevorzugt Tabletten oder alle Formen zum Lutschen und Kauen.

Beispiele:

1. Lutschtabletten

2500 g N-Acetylglucosmain werden mit 1000 g Lactose und 15 g Magnesiumstearat homogen vermischt, granuliert und unter hohem Druck zu Tabletten verpreßt. Einzelgewicht einer Tablette 351,5 mg Gehalt an N-Acetylglucosamin je Tablette: 250 mg Einnahme vor den Mahlzeiten, eine Tablette langsam im Munde zergehen lassen.

2. Lutschtabletten

2500 g N-Acetylglucosamin werden mit 250 g Zitronensäure und 100 g Polyvinylpyrrolidon homogen vermischt, mit 80 %igem Aethanol wenig angefeuchtet, zu Tabletten verpreßt und bei ca. 40 °C im Luftstrom getrocknet. Einzelgewicht je Tablette ca. 117,5 mg Gehalt an N-Acetylglucosamin je Tablette: 100 mg Einnahme von 1 bis 3 Tabletten morgens und abends, Tabletten nacheinander einnehmen, langsam im Munde zergehen lassen.

3. Granulat

2500 g N-Acetylglucosamin werden mit 100 g Ascorbinsäure zur Geschmacksverbesserung, 100 g Polyvinylpyrrolidon und 1000 g Maisstärke homogen vermischt, mit 80 % igem Aethanol zu einem Brei verrührt, granuliert und durch ein Sieb mit 0,5 bis 1 mm Maschenweite gesiebt. Ca. 150 mg des Granulates enthaltend ca. 100 mg N-Acetylglucosamin werden mit einem Meßbecher auf einen Löffel gegeben und jeweils einige Zeit vor den Mahlzeiten langsam im Mund augelöst. Es wird nicht mit Flüssigkeit nachgespült.

Figurenbeschreibung:

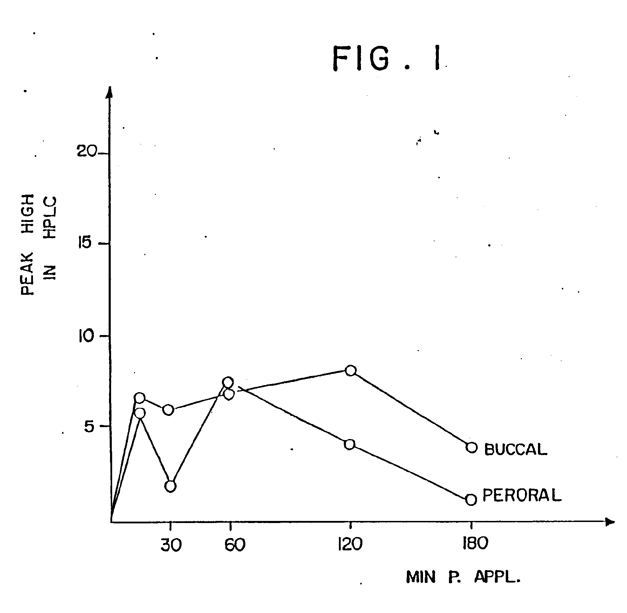

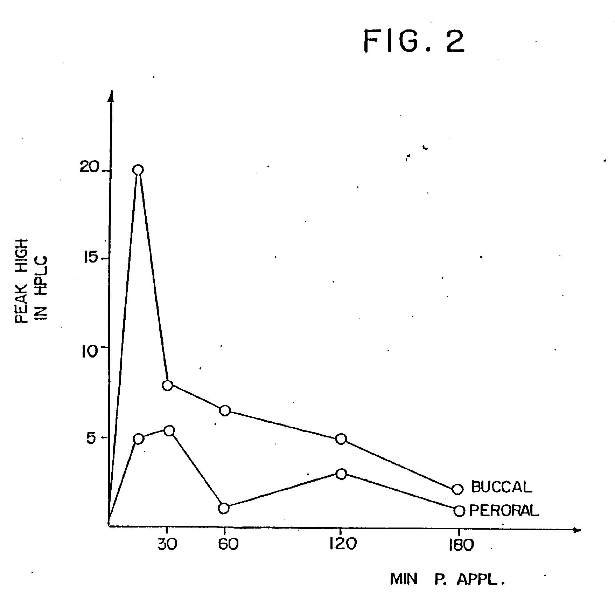

Fig. 1 vergleicht grafisch die Plasmapiegel bei Hamstern im Anschluß an intragastrale (perorale) und buccale Verabfolgung von 2 ml einer wäßrigen Lösung von 20 mg N-Acetylglucosamin. Fig. 2 vergleicht grafisch die Plasmapiegel bei Hamstern im Anschluß an intragastrale (perorale) und buccale Verabfolgung von 2 ml einer wäßrigen Lösung von 200 mg N-Acetylglucosamin.

**Patentansprüche**

1. Verwendung von N-Acetylglucosamin zur Herstellung eines buccal anzuwendenden Arzneimittels für die Therapie degenerativer Erkrankungen der Gelenke und des Binde- und Stützgewebes sowie verwandter Erkrankungen.

**2.** Verwendung von N-Acetylglucosamin zur Herstellung eines Arzneimittels nach Anspruch 1, dadurch gekennzeichnet, daß N-Acetylglucosamin in fester, halbfester oder flüssiger Form zusammen mit pharmazeutisch unbedenklichen Lösungs-, Träger- und/oder Hilfsstoffen engesetzt wird.

**3.** Verwendung von N-Acetylglucosamin zur Herstellung eines Arzneimittels nach Anspruch 1 oder 2, dadurch gekennzeichnet, daß N-Acetylglucosamin in fester Form als Pulver, Granulat, Tabletten, elastische oder plastische Kaumaterialien eingesetzt wird.

**4.** Verwendung von N-Acetylglucosamin zur Herstellung eines Arzneimittels nach einem der Ansprüche 1, 2 oder 3, dadurch gekennzeichnet, daß N-Acetylglucosamin unter Zusatz unbedenklicher und üblicher Geschmacksstoffe, Aromastoffe, pharmazeutischer Träger und Hilfsstoffe zur Stabilisierung, Formgebung und Steuerung der Freigabe eingesetzt wird.

**Claims**

**1.** Use of N-acetylglucosamine for the production of a pharmaceutical for buccal use for the therapy of degenerative disorders of the joints and of the connective and supporting tissue, as well as related disorders.

**2.** Use of N-acetylglucosamine for the production of a pharmaceutical according to Claim 1, characterised in that N-acetylglucosamine is employed in solid, semisolid or liquid form together with pharmaceutically acceptable solvents, vehicles and/or ancillary substances.

**3.** Use of N-acetylglucosamine for the production of a pharmaceutical according to Claim 1 or 2, characterised in that N-acetylglucosamine is employed in solid form as powder, granules, tablets, elastic or plastic chewable materials.

**4.** Use of N-acetylglucosamine for the production of a pharmaceutical according to any of Claims 1, 2 or 3, characterised in that N-acetylglucosamine is employed with the addition of acceptable and conventional flavourings, odorants, pharmaceutical vehicles and ancillary substances for stabilisation, moulding and controlling release.

**Revendications**

**1.** Utilisation de la N-acétylglucosamine dans la fabrication d'un médicament à utiliser par voie buccale pour le traitement de maladies dégénératives des articulations et du tissu conjonctif et protecteur, ainsi que de maladies apparentées.

**2.** Utilisation de la N-acétylglucosamine dans la fabrication d'un médicament selon la revendication 1, caractérisée en ce que la N-acétylglucosamine est utilisée sous forme solide, semi-solide ou liquide, conjointement avec des solvants, véhicules et/ou adjuvants pharmaceutiquement acceptables.

**3.** Utilisation de la N-acétylglucosamine dans la fabrication d'un médicament selon la revendication 1 ou 2, caractérisée en ce que la N-acétylglucosamine est utilisée à l'état solide sous forme de poudre, granulé, comprimés, matières élastiques ou plastiques à mâcher.

**4.** Utilisation de la N-acétylglucosamine dans la fabrication d'un médicament selon l'une des revendicaons 1, 2 ou 3, caractérisée en ce que la N-acétylglucosamine est utilisée avec addition de correcteurs de goût, arômes, véhicules et adjuvants pharmaceutiques, usuels et inoffensifs,"pour la stabilisation, la mise en forme et la régulation de la libération.

# FIG. 1

FIG. 2